# EUROPEAN PATENT SPECIFICATION

(11) **EP 2 611 501 B1**
(45) Date of publication and mention of the grant of the patent: **28.10.2020**
(21) Application number: 11822779.2
(22) Date of filing: 06.09.2011
(51) Int. Cl.: A61N 5/10, H05H 13/00, H05H 7/12, H05H 7/02, H01J 3/14

(54) **SYSTEM AND METHOD FOR AUTOMATIC CYCLOTRON PROCEDURES**
SYSTEM UND VERFAHREN FÜR AUTOMATISCHE ZYKLOTRONVERFAHREN
SYSTÈME ET PROCÉDÉ POUR DES PROCÉDURES AUTOMATIQUES DE CYCLOTRON

(30) Priority: 02.09.2011 US 201113224773; 03.09.2010 US 380017 P; 02.09.2011 US 201113225045
(43) Date of publication of application: 10.07.2013
(73) Proprietor: Varian Medical Systems Particle Therapy GmbH, 53842 Troisdorf (DE)
(72) Inventor: STEPHANI, Thomas, 51429 Bergisch Gladbach (DE); BEHRENS, Uwe, 21075 Hamburg (DE); ROECKEN, Heinrich, 53225 Bonn (DE); BAUMGARTEN, Christian, 5417 Untersiggenthal (CH); TIMMER, Jan, 50670 Cologne (FR)
(74) Representative: Kirchner, Veit
(86) International application number: PCT/US2011/050561
(87) International publication number: WO 2012/031299

(56) References cited:
- US-A1- 2009 236 545
- US-B1- 6 717 162
- A E Geisler ET AL: "COMMISSIONING OF THE ACCEL 250 MEV PROTON CYCLOTRON", , 1 October 2007 (2007-10-01), XP055123028, Retrieved from the Internet: URL:http://epaper.kek.jp/c07/PAPERS/9.PDF [retrieved on 2014-06-12]
- TIMMER J H ET AL: "Automated cyclotron tuning using beam phase measurements", NUCLEAR INSTRUMENTS & METHODS IN PHYSICS RESEARCH. SECTION A: ACCELERATORS, SPECTROMETERS, DETECTORS, AND ASSOCIATED EQUIPMENT, ELSEVIER BV * NORTH-HOLLAND, NL, vol. 568, no. 2, 1 December 2006 (2006-12-01), pages 532-536, XP027925997, ISSN: 0168-9002 [retrieved on 2006-12-01]
- Christina Wouters ET AL: "Central Region Studies of the 250 MeV SC Cyclotron for Proton Therapy", , 1 January 2010 (2010-01-01), XP055123046, Retrieved from the Internet: URL:https://www.kvi.nl/~agorcalc/ECPM2009/ Posters/02ECPM_wouters.pdf [retrieved on 2014-06-12]
- TIMMER, J. H. ET AL.: 'Automated cyclotron tuning using beam phase measureme nts' NUCLEAR INSTRUMENTS AND METHODS IN PHYSICS RESEARCH vol. 568, no. 2, 01 December 2006, pages 532 - 536
- GEISLER, A. E. ET AL.: 'COMMISSIONING OF THE ACCEL 250 MEV PROTON CYCLOTRON' CYCLOTRONS AND THEIR APPLICATIONS 2007, pages 9 - 14

## Description

### TECHNICAL BACKGROUND

Radiation therapy (RT) is a popular and efficient method for cancer treatment, where ionizing radiation is used in an attempt to destroy malignant tumor cells or to slow down their growth. RT is often combined with surgery, chemotherapy, or hormone therapy, but may also be used as a primary therapy mode. Radiation therapy is most commonly administered as external beam RT, which typically involves directing beams of radiated particles produced by sources located externally with respect to the patient or subject to the afflicted treatment area. The beam can consist of photons, electrons, protons or other heavy ions. As the beam travels through matter (e.g., the subject), energy from the ionizing radiation is deposited along the path in the surrounding matter. This energy is known as "dose," and is used to measure the efficacy and accuracy of a radiation beam. Malignant cells are damaged along the path of radiation beam during the RT. Unfortunately, the damage from the radiation is not limited to malignant cells and may be incurred by any interceding or adjacent cells. Thus, the dosage of radiation to healthy tissues outside the treatment volume is ideally minimized to avoid being collaterally damaged.

Proton therapy is one type of external beam radiation therapy, and is characterized for using a beam of protons to irradiate diseased tissue. The chief advantage of proton therapy over other particle-based therapies is the ability to administer treatment dosages three dimensionally, by specifying the *depth* (i.e., penetration) of applied radiation, thereby limiting the inadvertent exposure of un-targeted cells to the potentially harmful radiation. This enables proton therapy treatments to more precisely localize the radiation dosage when compared with other types of external beam radiotherapy. During proton therapy treatment, a particle accelerator, such as a cyclotron, is used to generate a beam of protons from an internal ion source located in the center of the cyclotron. Typically, a cyclotron is located in a location remote from the target treatment room. The generated protons are directed, via magnets, through a series of interconnecting tubes (called the beamline), and applied to a subject in a target treatment room.

A.E. Geisler et al. in "Commissioning of the ACCEL 250 MEV proton cyclotron", Cyclotrons and Their Applications 2007, EighteenthInternational Conference, present a fully operational proton cyclotron in clinical use and routine operation. J.H. Timmer et al. in "Automated cyclotron tuning using beam phase measurements", Journal of Nuclear Instruments and Methods in Physics research A 568 (2006) 532-536, describe the design of a beam phase detector and the effect of the phase regulation on the performance of a superconducting cyclotron.

Generally speaking,cyclotronsgenerate a proton beam at a fixed energy for the duration of a proton, therapy treatment. During typical proton radiation treatments however, irradiating a tumor often requires irradiating an entire volume (a tumor, for example) at different depths within a patient or treatment subject. These depths, which may be referred to in discrete units as layers, naturally correspond to different "optimal" energy levels. Since cyclotrons operate only at a fixed energy during a treatment session, irradiating different depths can become problematic. Conventional methods for irradiating a volume are performed by allying a single beam cummt,and begin by targeting the furthest depth within a patient or subject. For differing depths, a component (such as a carbon fitter or "degrader") is inserted into the path of the extracted beam at some distance from the cyclotron. The degrader material deduces the speed of the particles (and thereby the beam energy). Every time the proton beam's energy is changed this results in a new "layer" within the patient or target receiving treatment

However, the degrader material also reduces the density and number of particles comprising the beam (e.g., the "beam intensity") that continues past the degrader. In order to achieve proper dose rates for each layer, this may be compensated by increasing the beam intensity that the cyclotron delivers to the degrader input Unfortunately, using conventional techniques the change of beam intensity within a cyclotron can take a significant amount of time.

As a result, proton therapy according to conventional operating techniques is generally limited to relatively simple treatment plans, which even then may require exceptionally and inefficiently long irradiation times. In some cases, if the extracted beam current must be frequently varied according to a multiplicity of layers requiring treatment for example, new, practically separate treatment plans would need to be devised which would likely lead to even more delays and inefficiencies. Moreover, due to the complexity of the underlying machines, their operating and maintenance procedures, and the gravity of the corresponding medical procedures, highly trained and skilled operators are needed to perform the calculations and actions necessary to make adjustments to a proton beam current. Naturally, this can result in further inefficiency, delays or even potential hazards if qualified operators are not available.

Customarily, cyclotron maintenance procedures are performed during services and repairs. Additionally, due to its high consumptive energy usage during operation, a common practice is to shut down or keep a cyclotron in a reduced power state at the end of each day of operation. Conventionally, the re-initialization of cyclotrons after these customary overnight shut-downs or after a service action is completed was performed completely manually by an operator. Among the re-initialization procedures, the operator has to vary the magnetic field of the cyclotron by varying the current of the main coil within the cyclotron to generate a proton beam and to determine a stable working point of the cyclotron. Due to thermal drifts of the cyclotron iron, the magnet current must also be adjusted manually in regular intervals.

Additional readjustments of cyclotrons are also typically required after a service action or repair is performed. These readjustments typically include reconfiguring and rearranging moveable components within the cyclotron based on analyzed performance values. Generally, these readjustments are performed manually, and also require even well-trained technicians a considerable amount of time.

### SUMMARY

This Summary is provided to introduce a selection of concepts in a simplified form that is further described below in the Detailed Description. This Summary is not intended to identify key features or essential features of the claimed subject matter, nor is it intended to be used to limit the scope of the claimed subject matter. The invention is as defined in the appended claims.

According one aspect of the invention, a system and method are provided to automatically perform an efficient adjustment of beam currents generated by a proton therapy system within a wide range for each treatment layer. The proton therapy system may, for example, comprise a cyclotron or any other proton accelerator. In an embodiment, efficient adjustment is achieved by using beam current attenuation by an electrostatic vertical deflector installed in the inner center of the cyclotron. The beam current may, for example, be adjusted by applying a high voltage to the electrostatic vertical deflector. The protons will be deflected by the electrical field (generated by the applied voltage) to a vertical collimator. Due to the applied voltage, only a dedicated amount of protons will pass the vertical deflector system. At the beginning of each treatment the attenuation curve of the vertical deflector is recorded. Based on this attenuation curve, the vertical deflector voltage for the needed beam current of each irradiation layer is interpolated. With this procedure, the beam current could be automatically adjusted in minimal time over a wide range while maintaining a high level of precision.

According to an embodiment, a proton radiation treatment is prepared by first setting the maximum beam current to reach the nearest desired depth within the patient (e.g., by proper positioning of moveable phase slits in the cyclotron), subsequently, the beam current is attenuated by adjusting the voltage of an electrostatic vertical deflector to acquire a plurality of data points, thereby generating an interpolation table containing vertical deflector voltages versus resulting extracted beam currents. Using this interpolation table the appropriate vertical deflector settings are determined from the desired beam current setpoints, and adjustment of the extracted beam current can be performed automatically and rapidly, thereby advantageously extending the ability to provide more complex treatment plans while drastically reducing the overall irradiation time.

The maximum beam current for each patient treatment is adjusted by means of two movable phase slits based on an automatically generated look-up table. Beam current adjustment starts by setting the slit widths to defined values. The slits are then opened and allow a precise tuning of the maximal beam current. After the adjustment of the phase slits the dependency of the beam current on the voltage of the vertical deflector is recorded. According to some embodiments, the complete sequence can take approximately twenty seconds. Based on this suppression curve, the beam current can be subsequently changed within milliseconds over a wide dynamic range by changing the voltage setting of the vertical deflector during the irradiation.

According to an embodiment, a proton therapy system comprising a cyclotron is provided. The cyclotron may comprise, for example, an ion source for producing a proton treatment beam having a beam current; a plurality of beam path modulators (implemented as a plurality of electrostatic vertical deflectors with or without a plurality of vertical collimators); and a plurality of beam current modulators (implemented as, for example, a plurality of moveable phase slits). In a further embodiment, the proton treatment beam is automatically directed by the plurality of beam path modulators such that at least a portion of the proton treatment beam travels through the plurality of beam current modulators. The plurality of beam path modulators configurably directs at least a portion of the beam of protons through the plurality of beam current modulators by generating an electric field in the beam path modulators and causing a deflection in a trajectory of the beam of protons.

A plurality of extracted proton beams is subsequently generated from the proton treatment beam and applied to a plurality of target treatment layers disposed in a proton therapy patient. The plurality of target treatment layers may comprise, for example, target layers with varying depths in the proton therapy patient, and may further correspond to a treatment plan for the proton therapy patient. The plurality of extracted proton beams may be generated by attenuating the beam current of the proton treatment beam to correspond to the plurality of target treatment layers and by directing varying portions of the proton treatment beam through the plurality of beam current modulators according to pre-determined measurement data. In a further embodiment, the pre-determined measurement data consists of a data curve which plots a correspondence between a plurality of extracted beam currents generated from attenuating the beam current of the proton treatment beam and a voltage in the plurality of beam path modulators. Accordingly, a proton therapy treatment may be thus created which comprises a plurality of extracted beam currents corresponding to a plurality of target treatment layers generated by a proton system and applied to a proton therapy patient by automatically attenuating the beam current of the proton therapy treatment beam according to measurement data comprised in the data curve.

According to another embodiment, a method is provided for applying proton therapy in a cyclotron by: generating a beam of protons from an ion source, the beam of protons having a beam current; directing, via a plurality of beam path modulators, the beam of protons through a plurality of beam current modulators; conducting a plurality of beam current measurements by performing a plurality of beam current adjustments; analyzing the plurality of beam current measurements to determine a plurality of settings for the plurality of beam path modulators for producing a plurality of desired beam currents; and automatically producing the plurality of desired beam currents by adjusting a plurality of beam path modulators to conform to the plurality of pre-determined configurations. In a further embodiment, the pre-determined configurations correspond to a proton therapy treatment plan for a proton therapy patient.

Directing the beam of protons through a plurality of beam current modulators is performed by modifying openings of the beam current modulators. The plurality of desired beam currents is produced thereafter by, for example, adjusting a configuration of the plurality of beam path modulators to modify the trajectory of the beam of protons (by generating a voltage in the plurality of beam path modulators. In further embodiments, the trajectory of the beam of protons is modified such that only a portion of the beam of protons passes through the plurality of beam current modulators thereby producing an extracted beam of protons having a beam current corresponding to the portion of the beam of protons which passed through the plurality of beam current modulators.

Performing a plurality of beam current adjustments to conduct a plurality of beam current measurements is performed by configuring the plurality of beam path modulators according to the plurality of pre-determined configurations and measuring the beam currents of the proton beam produced. Analyzing the plurality of beam current measurements is performed by measuring beam currents of the proton beam produced when the plurality of beam path modulators is configured according to the plurality of pre-determined configurations and extrapolating the measured beam currents to generate a curve.

Automatically producing the plurality of desired beam currents is performed by receiving the plurality of desired beam currents corresponding to a plurality of target layers in the proton therapy patient; determining, on the generated curve, a plurality of beam path modulator configurations that correspond to the plurality of desired beam currents corresponding to the plurality of target layers in the proton therapy patient; and adjusting the plurality of beam path modulators to conform to the plurality of beam path modulator configurations that correspond to the plurality of desired beam currents. According to further embodiments, the plurality of target layers in a treatment target comprises a plurality of target layers with varying depths disposed in the proton therapy patient and corresponding to a proton therapy plan of the proton therapy patient.

According to another aspect of the invention, new techniques for automating initialization and calibration procedures of a proton therapy system (such as a cyclotron) are herein provided. A process is provided that allow the automation of the initialization of a cyclotron after overnight or maintenance imposed shutdown. In an embodiment, five independent cyclotron system states are defined and the transition between one state to another may be automated, e.g., by the control system of the cyclotron. According to these embodiments, it is thereby possible to achieve beam operation after shutdown with minimal manual input. By applying an automatic procedure, all active devices of the cyclotron (e.g., a radio-frequency "RF" sub-system, extraction deflectors, ion source, etc.) are respectively ramped to predefined parameters.

In an embodiment, automating the initialization procedures of a cyclotron may be performed by calculating the current set value of the main magnet of the cyclotron as a function of the cyclotron iron temperature. This allows a proton beam to be extracted out of the cyclotron directly at the end of the start-up procedure without the performance of any additional procedures. Additionally, the working point of the cyclotron is tuned by an automated phase control loop to guarantee the beam quality needed for medical operation of the system. The phase control loop tunes the magnetic field to an optimal level by analyzing and stabilizing the phase shift of the extracted beam with respect to the RF frequency of the cyclotron measured by a phase measurement system.

According to a third aspect of the invention, a system and methods are provided which allow calibration procedures to be automatically performed by the control system of the cyclotron to allow less skilled personnel to calibrate the cyclotron and to ensure the calibration is performed correctly.

These procedures may include performing beam centering by calculating the set values for the positions of the inner trim rods of the cyclotron by analyzing the radial behavior of the cyclotron's internal beam current measured with an internal straight beam probe or a radial beam probe. Another calibration procedure may include increasing the extraction efficiency by automatically analyzing the extracted beam current as influenced by the settings of the cyclotron's central chamber of components. Specifically, the outer trim rods or extraction trim coils, and the voltages applied to one or more extraction deflectors. Also disposed within a central chamber of a cyclotron are a plurality of phase slits used to modulate the beam current. To further improve a cyclotron's efficiency by reducing internal beam losses, the operative radial positions of the phase slits may also be automatically determined by analyzing the phase slit transmission curves of the cyclotron with respect to a defined position criteria. In an embodiment, each of these calibration steps may be performed automatically by the control system of the cyclotron. Thus, the operative settings can be determined without verification or oversight of highly skilled personnel.

According to an embodiment, a system is provided to perform automatic initialization and calibration of the proton therapy system. This system may be implemented as a proton therapy system, such as a cyclotron, which includes a plurality of sub-systems including, for example: a super-conducting magnet sub-system; a cryogenic-cooling sub-system; a vacuum sub-system; a radio frequency sub-system; and an ion source. Additional sub-systems may also include a vertical deflector system, a plurality of phase slits, an electrostatic extraction deflector sub-systems; a phase measurement system and an internal straight probe (and/or radial beam probe). According to such an embodiment, operation of the proton therapy system comprises a plurality of operation states corresponding to a plurality of activity levels of the plurality of sub-systems. The proton therapy system is configured to automatically traverse the plurality of operation states in sequence to activate the plurality of sub-systems in order prepare the proton therapy system to generate and extract a proton beam

Such a system may perform a process for automatic initialization of a proton therapy system. In an embodiment, such a process is performed for a proton therapy system by: automatically measuring a temperature of the magnet iron of the super-conducting magnet system in the proton therapy system; automatically determining a current configuration of a super-conducting magnet comprised in the proton therapy system based on the measured temperature; automatically setting a power supply of the super-conducting magnet to the calculated electrical current; generating a proton beam in an ion source comprised in the proton therapy system with the super-conducting magnet operating with the calculated electrical current; and extracting the generated proton beam. In alternate embodiments, the process further includes monitoring the extracted proton beam in a phase control loop by utilizing the signal of a non-destructive beam phase detector to measure the beam phase with respect to a radio frequency system of the cyclotron in a phase measurement system and quantifying effects of beam phase shifts due to incurred magnetic field drifts. In still further embodiments, the initialization process includes tuning a beam current stability of the proton therapy system by stabilizing the effect of the measured beam phase shifts through resetting the current in the super-conducting magnet to a desired (optimal) value corresponding to the measured beam phase shifts.

Such a system may also perform a process for automatic calibration of a proton therapy system. In an embodiment, such a process is performed by a control system of a proton therapy system by automatically centering a proton beam produced by the proton therapy system; automatically tuning an extraction efficiency of the proton beam produced by the proton therapy system; and automatically refining positions of a plurality of radial phase slits comprised in the proton therapy system. Automatically centering the proton beam is accomplished by measuring an oscillation of a current of the proton beam (by an internal straight probe, for example) to determine a precession of the proton beam; and reducing the precession of the proton beam. The precession of the proton beam is reduced by using a plurality of inner trim rods of the proton therapy system to adjust an amplitude and phase of the first harmonic of a magnetic field generated in the proton therapy system and corresponding to the oscillation of the current of the generated proton beam.

Automatically tuning the extraction efficiency is performed by analyzing a beam current of the proton beam and resetting values corresponding to a plurality of outer trim rods and voltages in a plurality of extraction deflectors of the proton therapy system to pre-defined values. For example, tuning the extraction efficiency of the proton therapy system may include exciting the precession of the proton beam prior to an extraction of the proton beam with the outer trim rods and/or adjusting the voltages in the plurality of extraction deflectors, the plurality of extraction deflectors configured to direct a trajectory of the extracted proton beam. Automatically refining positions for the plurality of radial phase slits in the proton therapy system is performed by determining a plurality of beam current measurements at the plurality of phase slits, the plurality of phase slits being positioned at a first plurality of radial positions; calculating a second plurality of radial positions based on the plurality of beam current measurements; and automatically re-positioning the phase slits at the second plurality of radial positions.

### BRIEF DESCRIPTION OF DRAWINGS

The accompanying drawings, which are incorporated in and form a part of this specification, illustrate embodiments of the invention and, together with the description, serve to explain the principles of the invention:
Figure 1 depicts a diagram of an exemplary proton accelerator, in accordance with various embodiments of the claimed subject matter.
Figure 2 depicts an expanded-view diagram of an exemplary proton accelerator, in accordance with various embodiments of the claimed subject matter.
Figure 3 depicts an illustration of a portion of a central chamber of an exemplary proton accelerator, in accordance with various embodiments of the claimed subject matter.
Figure 4 depicts an illustration of an example non-spiral trajectory without influence from an electric field generated by an electrostatic vertical deflector system in an exemplary proton accelerator, in accordance with embodiments of the claimed subject matter.
Figure 5 depicts an illustration of an example non-spiral trajectory influenced by an electric field generated by an electrostatic vertical deflector system in an exemplary proton accelerator, in accordance with embodiments of the claimed subject matter.
Figure 6 depicts a flowchart of a method of automatically generating multiple extracted beam currents in an exemplary proton accelerator, in accordance with embodiments of the claimed subject matter.
Figure 7 depicts a flowchart of a method of automatically initializing a proton accelerator, in accordance with embodiments of the claimed subject matter.
Figure 8 depicts a flowchart of a method of automatically calibrating a proton accelerator, in accordance with embodiments of the claimed subject matter.

### DETAILED DESCRIPTION

Reference will now be made in detail to several embodiments. While the subject matter will be described in conjunction with the alternative embodiments, it will be understood that they are not intended to limit the claimed subject matter to these embodiments. On the contrary, the claimed subject matter is intended to cover alternative, modifications, and equivalents, which may be included within the spirit and scope of the claimed subject matter as defined by the appended claims.

Furthermore, in the following detailed description, numerous specific details are set forth in order to provide a thorough understanding of the claimed subject matter. However, it will be recognized by one skilled in the art that embodiments may be practiced without these specific details or with equivalents thereof. In other instances, well-known methods, procedures, and components, have not been described in detail as not to unnecessarily obscure aspects and features of the subject matter.

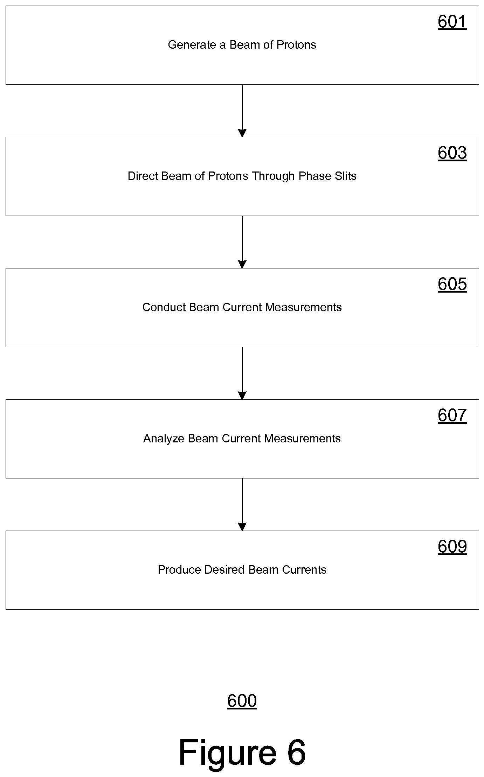

Portions of the detailed description that follows are presented and discussed in terms of a method. Although steps and sequencing thereof are disclosed in figures herein (e.g., Figure 6) describing the operations of this method, such steps and sequencing are exemplary. Embodiments are well suited to performing various other steps or variations of the steps recited in the flowchart of the figure herein, and in a sequence other than that depicted and described herein.

### EXEMPLARY PROTON ACCELERATOR

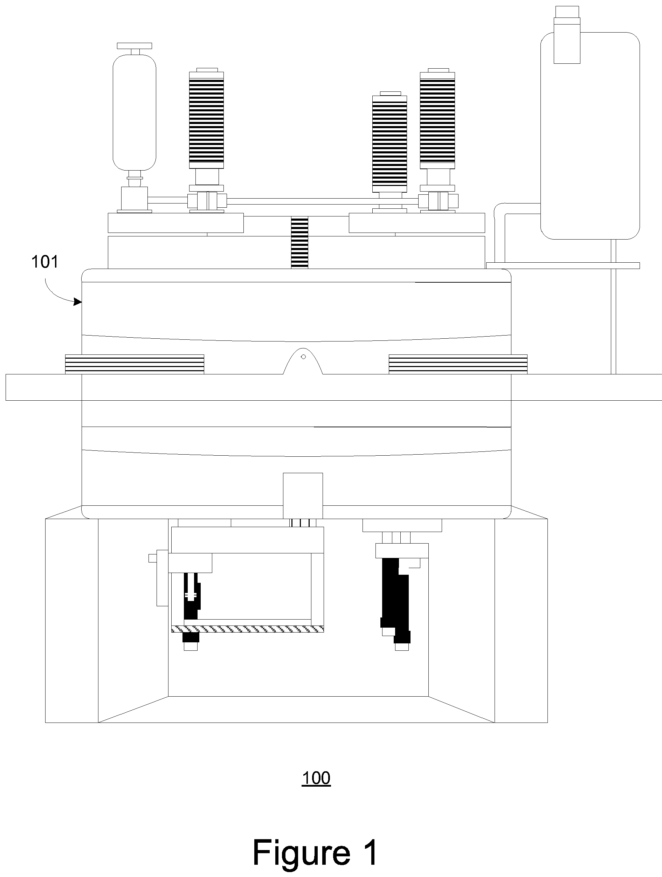

With reference now to Figure 1, an illustration of an exemplary proton accelerator 100 is depicted, in accordance with an embodiment. In one configuration, the cyclotron may be, for example, a compact four sector isochronous cyclotron incorporating a superconducting main coil. As depicted, the superconducting main coil may be housed in a central chamber 101, in which particles of a generated proton beam from an ion source are radially accelerated. In an exemplary configuration, the cyclotron operates with a beam energy of 250 MeV, and capable of generating a maximum beam current of about 800 nA and the typical extraction efficiency is 80%.

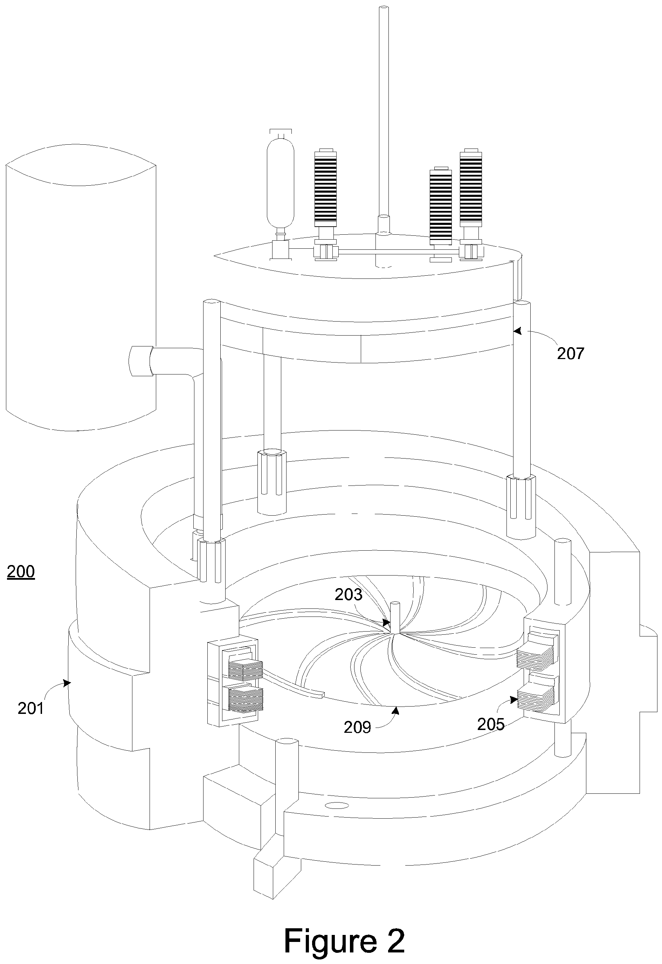

With reference now to Figure 2, an illustration of an expanded-view diagram of an exemplary proton accelerator 200, in accordance with various embodiments of the claimed subject matter. The exemplary proton accelerator 200 may, for example, be implemented as the cyclotron described above with reference to Figure 1. As depicted, Figure 2 depicts the upper portion of a cyclotron 200 with a raised top 207 (typical during the performance of maintenance, for example). A beam of protons is generated in a central chamber 209 by an ion source 203, and radially accelerated by electrical fields of a radio frequency (RF) sub-system and guided to the extraction radius in a spiral form by the magnetic field induced by the superconducting main coil (depicted, in part, as a portion of a main coil 205).

According to some embodiments, operation of a cyclotron to generate a proton beam for proton therapy may comprise a plurality of component sub-systems working in concert. These sub-systems may have dedicated functionalities and may include (but is not limited to): a super-conducting magnet sub-system, which includes the super-conducting magnet 205 and the control system thereof; a cryogenic-cooling sub-system and the control system thereof which maintains the thermal stability of the components of the cyclotron, such as the super-conducting magnet 205, and/or the temperature within the central chamber 209 of the cyclotron; a vacuum sub-system and the control system thereof for maintaining a pressure within the central chamber 209 of the cyclotron; a radio-frequency sub-system and the control system thereof for providing alternating electrical fields which accelerate the protons up to their target energy (e.g. 250 MeV), and an ion source and the control system thereof for generating a plurality of protons which comprises the proton beam.

In further embodiments, the plurality of component sub-systems also includes a vertical deflector plate sub-system, and the control system thereof, for generating an electric field that may be used to turn the beam on or off and to adjust the beam intensity of a generated proton beam. Additionally, the plurality of component sub-systems may also include a feedback or phase measurement sub-system and the control system thereof for monitoring a proton beam extracted from the cyclotron. According to some embodiments the feedback sub-system may be configured to measure and quantify the effect of beam phase shifts incurred by the proton beam due to drifts in the magnetic fields generated by the magnetic sub-system 205. Measurement and analysis of the beam phase shifts may be performed by, for example, a non-destructive beam phase detector disposed in the cyclotron. In still further embodiments, the feedback sub-system may also include a phase control loop configured to monitor and calibrate an extracted proton beam by, for example, tuning a magnetic field generated by the super-conducting magnet sub-system 205 system by measuring the effect of the beam phase shifts affecting the proton beam with respect to a radio frequency generated by the radio frequency sub-system during operation of the cyclotron. Additionally, the feedback sub-system may also perform tuning of the extraction efficiency and beam current stability for the proton beams produced by the cyclotron.

According to one embodiment, the component sub-systems each have an individual power state. These power states allow the sub-systems to be powered on, off, or operate at reduced power levels independent from each other. Powering off of one or more sub-systems may be required during maintenance, service, or daily shutdown procedures, for example. According to further embodiments, one or more of the component sub-systems may have power states that depend on the power state of another component sub-system, and therefore, powering a sub-system on (or off) may required being performed in a particular sequence. For example, an ion source may require the vacuum sub-system to be operational before the ion source is able to achieve an operational state capable of generating particles.

After an overnight or maintenance imposed shutdown, initialization of the cyclotron 200 may be performed. In one embodiment, a plurality of independent cyclotron system states are defined and the transition between one state to another in a sequence (which can be more than one particular sequence) is completely automated. The transition between the cyclotron system states may, for example, be performed according to an automatic procedure applied by the control system of the cyclotron 200. The automatic procedure may provide a sequence during which all active devices of the cyclotron (e.g., RF system, extraction deflectors, ion source) are respectively ramped to predefined parameters. According to these embodiments, it is thereby possible to achieve beam operation after shutdown with minimal manual input.

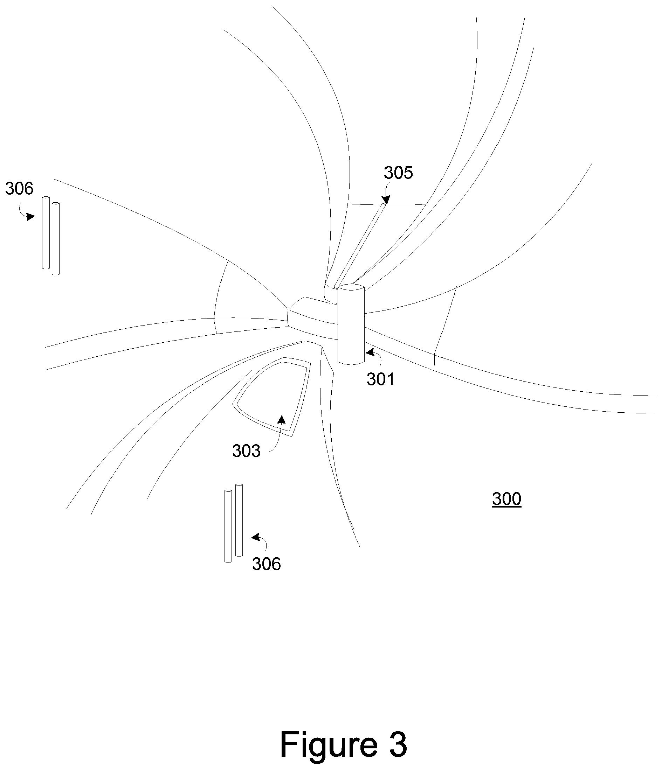

Figure 3 depicts a close-up illustration of a portion of the central chamber 300 (e.g., central chamber 209) in an exemplary proton accelerator (e.g., cyclotron 100 and 200), in accordance with various embodiments of the claimed subject matter. The portion of the central chamber 300 depicted in Figure 3 includes an internal ion source 301, an electrostatic vertical deflector plate 303, a vertical collimator plate 305. According to an embodiment, during a proton therapy treatment session, a proton beam emanates as a stream or burst of protons from the ion source 301, the stream (or burst) of protons is accelerated by electrical fields generated by a radio frequency (RF) sub-system of the cyclotron and guided to the extraction radius in a spiral form via the magnetic field in the central chamber 300 produced by magnetic coils (e.g., magnetic coils 205).

The produced proton beam is threaded through one or more phase slits (e.g., phase slit 306), resulting in an extracted proton beam having a corresponding beam current. As presented, the phase slits 306 include apertures through which the proton beam travels. In an embodiment, the size of the aperture of a phase slit 306 is of sufficient size to allow a generated proton beam to traverse completely unimpeded. According to alternate embodiments, the size of the aperture of the phase slit 306 is less than that of a complete proton beam, such that at least a portion of any generated proton beam is absorbed by the material of the phase slit 306 when the proton beam is travelling through the aperture.

Tuning the proton beam (and by extension, the beam current of an extracted beam) may be performed by producing a voltage in the electrostatic vertical deflector 303, thereby producing an electric field. The properties imparted by the electric field affect the particles comprised in the beam of protons, and is capable of influencing the trajectory of the produced proton beam. A stronger electric field produces a greater influence, and thus, the proton beam is capable of being aimed to the extent that the trajectory of the proton beam is at least partially directed into (and thus, intercepted by) the vertical collimator 305 by running varying voltages through the electrostatic vertical deflector 303. For example, in an embodiment, a maximum beam current may correspond to little or no voltage in the electrostatic vertical deflector 303, thereby leaving most if not all of the produced beam through the aperture unimpeded.

The transmitted beam will be guided by the vertical deflector and collimator sub-systems to the phase slits 306. According to an embodiment, the opening widths of the phase slits are variable and a portion of the proton beam could be stopped by this measure. If the apertures of the phase slits are completely open, the transmitted beam guided by the vertical deflector and collimator sub-systems and the extracted beam may have approximately the same beam current (only influenced by the extraction efficiency of the cyclotron). The magnitude of the electric field of the vertical deflector may be controlled by applying smaller or larger voltages to the electrostatic vertical deflector 303. The portion of the proton beam that travels through the apertures of the vertical collimator and the phase slits (the "extracted" beam) has a decreased beam current with an intensity lowered by an amount which corresponds to the portion of the produced proton beam intercepted by the non-aperture portion of the vertical collimator and the phase slits.

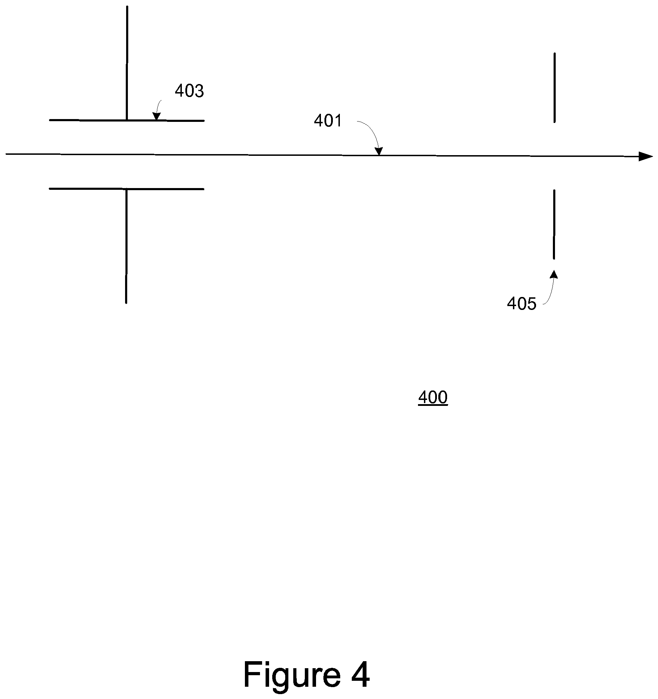

With reference now to Figure 4, an illustration 400 of an example trajectory 401 of a proton beam without being influenced by an electric field generated by a electrostatic vertical deflector 403 in an exemplary proton accelerator is depicted, in accordance with embodiments of the claimed subject matter. As presented in Figure 4, an electrostatic vertical deflector 403 and a vertical collimator 405 with an aperture are disposed in a cyclotron (e.g., cyclotron 100 and 200). In further embodiments, the electrostatic vertical deflector 403 and vertical collimator 405 are disposed within a central chamber (e.g., central chamber 209) of the cyclotron. A generated proton beam (e.g., emanating from an ion source such as ion source 203) with sample trajectory 401 travels through an area adjacent to an electrostatic vertical deflector 403, and towards (and through) the vertical collimator 405. As shown, with little (or no) influence by an electric field generated by the electrostatic vertical deflector 403, the generated proton beam is able to travel directly through the aperture of the vertical collimator 405 unimpeded, losing little to no beam intensity (and thus, maintaining a consistent beam current) in the process. As presented, the sample trajectory 401 may, for example, correspond to a maximum desired beam current for a proton treatment therapy.

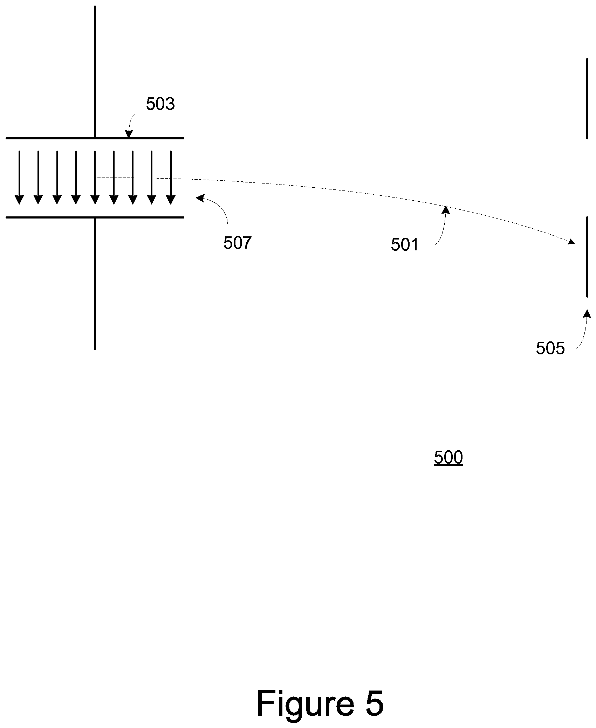

Conversely, and with reference now to Figure 5, an illustration 500 of an example trajectory 501 of a proton beam influenced by an electric field generated by a electrostatic vertical deflector 503 in an exemplary proton accelerator is depicted, in accordance with embodiments of the claimed subject matter. As presented in Figure 5, the electrostatic vertical deflector 503 and vertical collimator 505 with an aperture and corresponding to their equivalents in Figure 4 (electrostatic vertical deflector 403 and a vertical collimator 405, respectively) are disposed within a central chamber (e.g., central chamber 209) of a cyclotron (e.g., cyclotron 100 and 200). According to an embodiment, running a voltage through the electrostatic vertical deflector 503 creates an electric field 507. A generated proton beam (e.g., emanating from an ion source such as ion source 203) with sample trajectory 501 travelling through the electric field 507 towards (and through) vertical collimator 505 will be impacted by the properties of the electric field 507. An electric field with sufficient magnitude is capable of altering the trajectory of the proton beam. As shown, with sufficient influence by an electric field generated by the electrostatic vertical deflector 503. the generated proton beam can be directed towards the non-aperture portion of the vertical collimator 505, having the particles of the beam impeded by the non-aperture portion of the vertical collimator 505 being absorbed by the material of the vertical collimator 505, and losing beam intensity proportional to the portion of the beam that was obstructed (and thus, decreasing the beam current) in the process. As presented, the sample trajectory 501 may, for example, correspond to a desired beam current less than the maximum beam current (e.g., shallower "layers" in a target subject) for a proton treatment therapy.

### PROTON BEAM TRAJECTORY TUNING

During medical operation, the control and oversight of a generated proton beam may be completely managed by a higher level control system operating in a computer system (thus not necessitating manual operation). This management may include, for example, the requests for desired beam currents. During operation of the cyclotron, adjustments to the generated beam may be performed to produce varying extracted beams. According to an embodiment, a proton radiation beam is generated at step 601. The generated proton radiation beam may have an initial beam current set to the maximum beam current calculated to reach the needed intensity for the lowest desired depth for a radiation treatment plan (e.g., by proper positioning of moveable phase slits in the cyclotron). The maximum beam current for each treatment is adjusted by means of two movable phase slits (the position and/or aperture size of phase slits may, for example, be calculated by manually or automatically referencing a table of configuration data, for example). Beam current adjustment may begin by setting the phase slit widths to pre-defined values. The slits are then opened to allow passage of the generated beam at step 603, and precise tuning of the beam current (e.g., typically within the range between 1 nA to 800 nA) may be performed by adjusting the positions of the phase slits.

The tuning of the beam current is performed by a multi-step process which begins by measuring extracted beam currents under varying circumstances, performed at step 605. After the adjustment of the phase slits, varying voltages are generated in a vertical deflector, producing electric fields of varying magnitudes. The generated electric fields influence the trajectory of the produced beam traveling through the vertical collimator. For example, if the magnitude of the field is sufficient to affect the trajectory such that a portion of the proton beam's path is obstructed by the non-aperture portion of the vertical collimator, the resultant beam current is attenuated by a corresponding amount. The resulting suppressed beam current is monitored and tracked for varying pre-determined data points (e.g., the radial positions of the phase slits, the voltage in the vertical deflector), and the correspondence of the extracted beam and the voltage of the vertical deflector is measured and recorded (e.g., at a local or remotely located computing device). According to some embodiments, five or more data points (e.g., configurations) may be tracked and monitored for a treatment during the tuning process. In further embodiments, the five or more data points may be distributed in the range between the maximum desired beam current and the minimum desired beam current for a proton treatment therapy session.

The data is then analyzed at step 607 to determine the correspondence between an extracted beam current and a voltage of the vertical deflector required to produce a beam having the extracted beam current (e.g., by extrapolating the data points to approximate a curve). The complete sequence may, according to some embodiments, take up to twenty seconds. Once the data points are analyzed and the appropriate vertical deflector positions are determined, the adjustment of the beam to produce extracted beams with varying beam currents (and thus, intensity) can be performed automatically and rapidly. Based on this plotted curve, the beam current can be subsequently adjusted within milliseconds (e.g., 600 ms), such that the beam currents corresponding to various layers in a treatment plan for a patient - that is, the depths desired within a target subject according to a proton treatment plan - can be generated by determining, on the analyzed suppression curve, the configurations of the electrostatic vertical deflector which correspond to the desired beam currents. Thus, once steps 601-607 are performed, a proton treatment plan comprised of multiple layers in a target patient may be treated with a single generated proton beam by automatically producing varying extracted beams with beam currents corresponding to each of the layers over a wide dynamic range by changing the voltage setting of the vertical deflector to allow efficient, versatile, and adjustable proton radiation treatments.

### AUTOMATED INITIALIZATION OF SUPERCONDUCTING PROTON CYCLOTRON

After an overnight or maintenance imposed shutdown, initialization of a cyclotron may be required in order for the cyclotron to achieve operational levels. The transition between the cyclotron system states may, for example, be performed according to an automatic sequence or cycle and applied by the control system of the cyclotron. Each cyclotron system state corresponds to a frequently used system state, and defines the operational (or "power") state and activity level of each sub-system of the cyclotron's operation. The automatic procedure may provide a sequence during which all active devices of the cyclotron (e.g., RF system, extraction deflectors, ion source) are respectively ramped to predefined parameters. According to these embodiments, it is thereby possible to achieve beam operation after shutdown with minimal manual input.

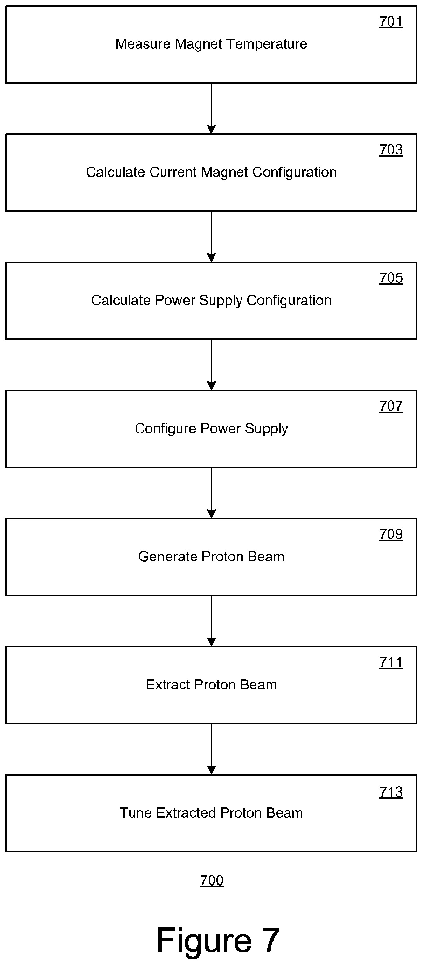

Figure 7 depicts an example procedure 700 wherein five independent cyclotron system states are defined and the transition between one state to another in sequence is partially or completely automated. The transition between these states may be performed to initialize a cyclotron in preparation for operation, or to variably shutdown or deactivate all or portions of the cyclotron in anticipation of a overnight shutdown, service procedure, or lengthy deactivation. Steps 701-713 describe exemplary steps of the process 700 in accordance with the various embodiments herein described.

In one embodiment, the five cyclotron states may be characterized as "Off"; "Standby 1"; "Standby 2"; "RF Ready"; and "Beam Ready." The cyclotron state "Off" may be implemented to completely deactivate (power off) all component sub-systems of the cyclotron except for the cryogenic systems, and may be used when the cyclotron must be opened and is non-operational, such as during a service routine. In alternate embodiments, the cyclotron state "Off" may be implemented to deactivate all component sub-systems except for the cryogenic cooling sub-systems. The cyclotron state "Standby 1" may be implemented to evacuate the cyclotron using the vacuum control system, but de-activate (or maintain deactivation of) the remaining sub-systems of the cyclotron. In further embodiments, the super-conducting magnet sub-system may operate in a reduced power state during the cyclotron state Standby 1. Standby 1 may be used, for example, when the cyclotron is closed and evacuated, such as when longer shutdown periods are expected. Transitioning between the Off and Standby 1 cyclotron states (and vice versa) may be performed automatically through the control terminal of the cyclotron.

Cyclotron state Standby 2 may be used during overnight shutdowns, such as at the end of daily operation to reduce the consumption of energy usage. During Standby 2, the cryogenic-cooling sub-system, the super-conducting magnet sub-system, and the vacuum sub-system are active with the remaining sub-systems deactivated and non-operational. To maintain the thermal stability of the magnet iron and other cyclotron parts, the cooling water of various sub-systems may be set to an increased temperature between Standby 1 and Standby 2. This also has the effect of minimizing transient effects caused by heating from radio frequency losses when returning to standard beam operation. Starting from Standby 2, beam operation may be possible within minutes, according to embodiments of the claimed subject matter. With the automated transition to the cyclotron state RF Ready, the radio frequency amplifier is switched on and the RF sub-system is set to operation. Conversely, when transitioning from RF Ready to Standby 2, the radio frequency amplifier is switched off and the RF sub-system is deactivated. In one embodiment the RF sub-system operates at a reduced power state in the cyclotron state RF Ready, and may be used as an alternate overnight shutdown state, or during short standby periods (e.g., between procedures). In further embodiments, the reduced power state of the RF sub-system operates at about 50% of its maximum power. A subsequent transition to the state Beam Ready sets the remaining components (e.g., ion source, extraction deflectors, vertical deflector and feedback sub-systems) to pre-defined default values as well as increasing the power state of the RF sub-system to a standard beam ready state.

An automatic initialization procedure may be performed by automatically cycling through the cyclotron states in sequence, such as the sequence according to process 700 of Figure 4. According to process 700, the temperature of a super-conducting magnet in the super-conducting magnet sub-system of a cyclotron is measured at step 701. The temperature of the super-conducting magnet may be measured by, for example, measuring the temperature of the iron comprising the super-conducting magnet. According to alternate embodiments, the temperature of various components of the super-conducting magnet (e.g., the magnet coils 205) may be measured. Once the temperature of the cyclotron iron is determined, the current configuration of the main magnet of the cyclotron is calculated at step 703 as a function of the measured iron temperature. A configuration of the power supply required to bring the cyclotron to standard operational state is then determined at step 705. For example, a low iron temperature measured in step 701 corresponds to a reduced current setpoint of the magnet. If such a current setpoint is calculated, the power-supply may be instructed to reduce the current of the cyclotron magnet, automatically bringing the super-conducting magnet sub-system to standard operational settings. Conversely, if the iron temperature already corresponds to standard operational temperatures, no change may be performed in the power-supply configuration. The power-supply of the super-conducting magnet is configured to correspond to the required configuration to bring the cyclotron to standard operation at step 707. Once the super-conducting magnet sub-system is fully powered after step 707, a proton beam is generated by the ion source at step 709. Once the beam is generated, the proton beam may be extracted out of the cyclotron at step 711 by configuring the beam path of the generated beam. Configuring the beam path of the generated beam may comprise, for example, generating an electric field which directs (or deflects) the trajectory of the beam by applying only a low voltage in the electrostatic vertical deflectors of the vertical deflector sub-system. Accordingly, an extraction of a proton beam from any of the plurality of cyclotron states may be achieved by performance of steps 701-711, directly at the end of the start-up procedure and without the performance of any additional procedures.

In further embodiments, the working point (operational settings) of the cyclotron is tuned by an automated phase control loop at step 713 to ensure the beam quality needed for medical operation of the system. The phase control loop tunes the magnetic field to an operational level by, for example, monitoring an extracted beam in the phase control loop and further tuning the monitored, extracted beam as necessary to conform to a desired configuration. Monitoring the extracted beam may comprise, for example, measuring the time behavior (phase) of the extracted beam with respect to the radio frequency system of the cyclotron in a feedback sub-system or phase measurement system. The phase of the extracted beam may be measured by a non-destructive beam phase detector, for instance. By monitoring the extracted beam, the effects of phase shifts of an extracted beam caused by magnetic field drifts or other influences may be measured and quantified with respect to the RF frequency of the cyclotron. Once the extracted beam is monitored for the effects of phase shifts, the extracted beam may be further tuned to stabilize the effect of the beam phase shifts by resetting the current in the super-conducting magnet to a desired value.

### AUTOMATED TUNING PROCEDURES FOR SUPERCONDUCTING PROTON CYCLOTRON

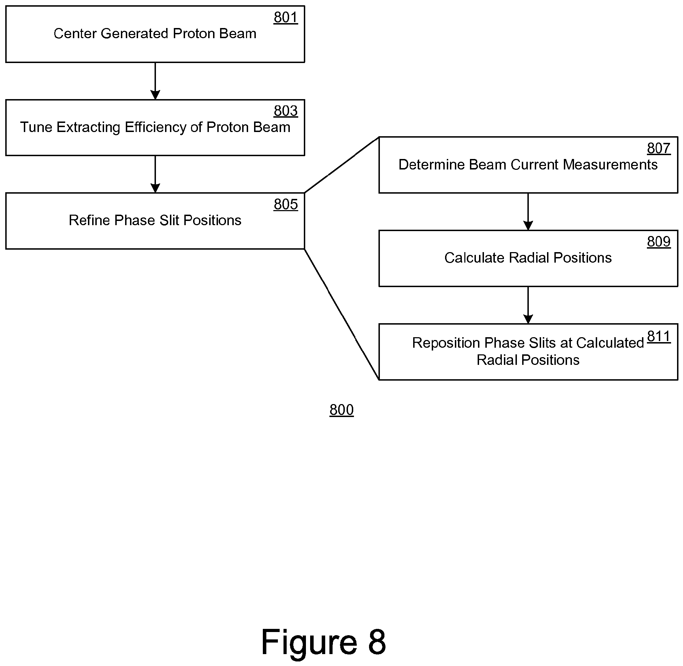

After a service action or maintenance routine, a cyclotron may require re-tuning in order to restore an operational configuration of the cyclotron. For example, a service action may cause a displacement or resetting of one or more values or configurations of the various sub-systems and sub-system components to lie outside an operational or desired range. This may cause a generated proton beam with a reduced extraction efficiency due to unintentional variations in the positioning of trim rods inside a cyclotron resulting from the performance of a service action or maintenance routine. Other sensitive components such as electrostatic deflectors and phase slits may be similarly impacted during these routines. Figure 8 depicts an example procedure 800 for automated tuning procedures of a proton cyclotron. The procedure 800 may be performed, for example, after a service action or maintenance routine which requires the removal and/or replacement of physical, hardware components of the cyclotron, for example. Additionally, the procedure 800 may be performed after long periods of inactivity, or to correct a sub-optimal performance of the cyclotron. Steps 801-811 describe exemplary steps of the process 800 in accordance with the various embodiments herein described.

According to process 800, a generated proton beam is automatically centered at step 801. Centering a generated proton beam may be performed by, for example, proper positioning of a plurality of moveable trim rods disposed in the center of the cyclotron. These trim rods are known as the inner trim rods (to be distinguished from outer trim rods placed at the radius of the extraction point) of the cyclotron. In one embodiment, the inner trim rods may be comprised of eight iron trim rods. Automatically centering the generated proton beam may be performed by measuring an oscillation of the beam current to determine the precession of the generated proton beam. Measuring the oscillation may be performed with an internal straight probe or a radial beam probe, for example. Once the oscillation is measured, the proton beam may be centered by using the plurality of inner trim rods to adjust the amplitude and phase of the first field harmonic of the generated magnetic field changes to reduce the precession of the generated beam. According to further embodiments, a radial beam probe is used to perform multiple probe scans and calculates proper inner trim rod settings (e.g., positions) based on the data from the scans. Once the settings are determined, the inner trim rods may be automatically positioned to center the proton beam at step 801.

At step 803, an extraction efficiency of the generated proton beam is tuned. The extraction efficiency may be tuned by analyzing the beam current of the extracted beam with respect to the position of the outer trim rods (e.g., the set of movable trim rods placed at the radius of the extraction point in the cyclotron) and the voltages of the one or more electrostatic deflectors ("extraction deflectors") and resetting the position of the outer trim rods and/or voltage of the extraction deflectors to pre-defined values. According to one embodiment, the extraction efficiency may be further tuned by exciting the precession of the generated proton beam prior to the extraction of the proton beam to increase the turn separation of the particles in the proton beam at the electrostatic deflectors. Exciting the precession of the generated proton beam may be performed by positioning of the outer trim rods, for example.

At step 805, the positions of a plurality of radial phase slits (e.g., phase slits 306) are refined. The positions of the plurality of radial phase slits 306 may be refined by, for example, determining a plurality of beam current measurements taken at the plurality of phase slits at a first radial position (at optional step 807), calculating a preferred or better performing configuration of the plurality of phase slits at a second radial position (at optional step 809), and automatically re-positioning the plurality of phase slits to the second radial position (at optional step 811). By re-positioning the plurality of phase slits to a second, better performing radial position, internal beam losses can be reduced.

By using the automatic procedures described above for the initialization and tuning of a cyclotron, operation of a cyclotron from an off or reduced power state to a fully configured, operational state may be performed with greatly improved efficiency, and requiring only minimal oversight.

Although the subject matter has been described in language specific to structural features and/or processological acts, it is to be understood that the subject matter defined in the appended claims is not necessarily limited to the specific features or acts described above. Rather, the specific features and acts described above are disclosed as example forms of implementing the claims. The embodiments, aspects and examples which do not fall within the scope of the claims are provided for illustrative purpose only and do not form part of the present invention. The invention is defined in the claims as follows:

## Claims

1. A method (600) for generating a beam of protons and performing an adjustment of beam currents by:
generating a beam of protons from an ion source (203, 301), the beam of protons having a beam current;
directing, via a plurality of beam path modulators, the beam of protons through a plurality of beam current modulators;
conducting a plurality of beam current measurements by performing a plurality of beam current adjustments by varying voltages of the beam path modulators;
analyzing the plurality of beam current measurements to determine a correspondence between the beam current measurements and the voltages of the beam path modulators;
determining a plurality of configurations for the plurality of beam path modulators using the correspondence between the beam current measurements and the voltages of the beam path modulators, for producing a plurality of desired beam currents; and
automatically producing the plurality of desired beam currents by adjusting the beam path modulators to conform to the plurality of configurations.

2. The method according to claim 1, wherein the plurality of configurations comprise a plurality of voltage levels that correspond to the plurality of desired beam currents.

3. The method according to claim 1 or claim 2, wherein the generating, the directing, the conducting, the analyzing and the producing are performed in a cyclotron (100, 200).

4. The method according to anyone of claims 1 to 3, wherein the plurality of beam path modulators comprises a plurality of vertical deflector plates (303, 403, 503).

5. The method according to according to anyone of claims 1 to 4, wherein the plurality of beam current modulators comprises a plurality of moveable phase slits (306).

6. Method according to claim 3, for automatic cyclotron initialization comprising:
automatically measuring a temperature of a plurality of components in a superconducting magnet comprised in the cyclotron;
automatically determining a current configuration of the superconducting magnet of the cyclotron based on the temperature;
automatically setting a super-conducting magnet power supply of the cyclotron to the determined current configuration;
generating the beam of protons in the ion source comprised in the cyclotron with the superconducting magnet operating with the determined current configuration; and
extracting the beam of protons.

7. The method of claim 6 further comprising monitoring the extracted beam of protons in a phase control loop.

8. The method of claim 7 further comprising tuning the extracted beam of protons monitored in the phase control loop.

9. Method according to claim 3, for automatically calibrating operation of the cyclotron, the method comprising:
automatically centering the beam of protons produced by the cyclotron;
automatically tuning an extraction efficiency of the beam of protons produced by the cyclotron; and
automatically refining positions of a plurality of radial phase slits comprised in the cyclotron,
wherein the centering, the tuning and the refining are performed automatically by a control system of the cyclotron.

10. The method according to claim 9 wherein automatically centering the beam of protons comprises:
measuring an oscillation of a current of the beam of protons to determine a precession of the beam of protons; and
reducing the precession of the beam of protons.

11. The method according to claim 10, wherein the cyclotron comprises a first plurality of trim rods, and wherein reducing the precession of the beam of protons comprises using the first plurality of trim rods to adjust an amplitude and a phase of a first harmonic of the generated magnetic field corresponding to the oscillation of the current of the beam of protons.

12. The method according to anyone of claims 9 to 11, wherein automatically tuning an extraction efficiency comprises analyzing the beam current of the beam of protons and resetting values corresponding to a second plurality of trim rods and voltages in a plurality of extraction deflectors to pre-defined values.

13. The method according to anyone of claims 9 to 12, wherein the automatically refining positions for the plurality of radial phase slits comprises:
determining a plurality of beam current measurements at the plurality of phase slits, the plurality of phase slits being positioned at a first plurality of radial positions;
calculating a second plurality of radial positions based on the plurality of beam current measurements at the phase slits; and
automatically re-positioning the phase slits at the second plurality of radial positions.

14. The method of anyone of claims 6 to 13, wherein the method is performed by the control system of the cyclotron, the cyclotron comprising a plurality of sub-systems including:
a super-conducting magnet sub-system;
a cryogenic-cooling sub-system;
a vacuum sub-system;
a radio frequency sub-system;
a vertical deflector sub-system,
the plurality of phase slits,
the plurality of electrostatic extraction deflectors,
a radial beam probe,
a phase measurement sub-system, and
the ion source;
wherein an operation of the cyclotron comprises a plurality of operation states, the plurality of operation states corresponding to a plurality of power states of the plurality of subsystems,
wherein the cyclotron is configured to at least partially automatically perform the plurality of operation states in sequence to prepare the cyclotron to generate and extract the beam of protons.

15. The method of claim 14, wherein the plurality of operation states activate the plurality of sub-systems in a predefined sequence.

## Patentansprüche

1. Verfahren (600) zum Erzeugen eines Protonenstrahls und zum Durchführen einer Anpassung von Strahlströmen durch:
Erzeugen eines Protonenstrahls aus einer lonenquelle (203, 301), wobei der Protonenstrahl einen Strahlstrom besitzt;
Lenken des Protonenstrahls über eine Vielzahl von Strahlwegmodulatoren durch eine Vielzahl von Strahlstrommodulatoren;
Ausführen einer Vielzahl von Strahlstrommessungen durch Durchführen einer Vielzahl von Strahlstromanpassungen durch Variieren von Spannungen der Strahlwegmodulatoren;
Analysieren der Vielzahl von Strahlstrommessungen zum Bestimmen einer Entsprechung zwischen den Strahlstrommessungen und den Spannungen der Strahlwegmodulatoren;
Bestimmen einer Vielzahl von Konfigurationen für die Vielzahl von Strahlwegmodulatoren unter Verwendung der Entsprechung zwischen den Strahlstrommessungen und den Spannungen der Strahlwegmodulatoren zum Erzeugen einer Vielzahl von erwünschten Strahlströmen; und
automatisches Erzeugen der Vielzahl von erwünschten Strahlströmen durch Anpassen der Strahlwegmodulatoren, so dass sie der Vielzahl von Konfigurationen entsprechen.

2. Verfahren nach Anspruch 1, wobei die Vielzahl von Konfigurationen eine Vielzahl von Spannungspegeln aufweist, die der Vielzahl von erwünschten Strahlströmen entspricht.

3. Verfahren nach Anspruch 1 oder Anspruch 2, wobei das Erzeugen, das Lenken, das Ausführen, das Analysieren und das Produzieren in einem Zyklotron (100, 200) durchgeführt werden.

4. Verfahren nach einem der Ansprüche 1 bis 3, wobei die Vielzahl von Stromwegmodulatoren eine Vielzahl von vertikalen Ablenkplatten (303, 403, 503) aufweist.

5. Verfahren nach einem der Ansprüche 1 bis 4, wobei die Vielzahl von Strahlstrommodulatoren eine Vielzahl von bewegbaren Phasenschlitzen (306) aufweist.

6. Verfahren nach Anspruch 3 zur automatischen Zyklotron-Initialisierung, umfassend:
automatisches Messen einer Temperatur einer Vielzahl von Bauelementen in einem im Zyklotron enthaltenen supraleitenden Magneten;
automatisches Bestimmen einer aktuellen Konfiguration des supraleitenden Magneten des Zyklotrons auf Basis der Temperatur;
automatisches Einstellen einer Energieversorgung des supraleitenden Magneten des Zyklotrons auf die bestimmte aktuelle Konfiguration;
Erzeugen des Protonenstrahls in der lonenquelle, die im Zyklotron enthalten ist, mit dem mit der bestimmten aktuellen Konfiguration arbeitenden supraleitenden Magneten; und
Extrahieren des Protonenstrahls.

7. Verfahren nach Anspruch 6, welches ferner das Überwachen des extrahierten Protonenstrahls in einer Phasenregelschleife umfasst.

8. Verfahren nach Anspruch 7, welches ferner das Abstimmen des extrahierten Protonenstrahls, der in der Phasenregelschleife überwacht wird, umfasst.

9. Verfahren nach Anspruch 3 zum automatischen Kalibrieren des Betriebs des Zyklotrons, wobei das Verfahren umfasst:
automatisches Zentrieren des durch das Zyklotron produzierten Protonenstrahls;
automatisches Abstimmen einer Extraktionseffizienz des vom Zyklotron produzierten Protonenstrahls; und
automatisches Feineinstellen der Positionen einer Vielzahl von radialen Phasenschlitzen, die im Zyklotron enthalten sind,
wobei das Zentrieren, das Abstimmen und das Feineinstellen von einem Steuersystem des Zyklotrons automatisch durchgeführt werden.

10. Verfahren nach Anspruch 9, wobei das automatische Zentrieren des Protonenstrahls umfasst:
Messen einer Schwingung eines Stroms des Protonenstrahls zum Bestimmen einer Kreiselbewegung des Protonenstrahls; und
Reduzieren der Kreiselbewegung des Protonenstrahls.

11. Verfahren nach Anspruch 10, wobei das Zyklotron eine erste Vielzahl von Trimmstäben aufweist und wobei das Reduzieren der Kreiselbewegung des Protonenstrahls das Verwenden der ersten Vielzahl von Trimmstäben zum Anpassen einer Amplitude und einer Phase einer ersten Harmonischen des erzeugten Magnetfelds entsprechend der Schwingung des Stroms des Protonenstrahls umfasst.

12. Verfahren nach einem der Ansprüche 9 bis 11, wobei das automatische Abstimmen einer Extraktionseffizienz das Analysieren des Strahlstroms des Protonenstrahls und das Zurücksetzen von Werten, die einer zweiten Vielzahl von Trimmstäben und Spannungen in einer Vielzahl von Extraktionsablenkern entsprechen, auf vordefinierte Werte umfasst.

13. Verfahren nach einem der Ansprüche 9 bis 12, wobei das automatische Feineinstellen von Positionen für die Vielzahl von radialen Phasenschlitzen umfasst:
Bestimmen einer Vielzahl von Strahlstrommessungen an der Vielzahl von Phasenschlitzen, wobei die Vielzahl von Phasenschlitzen an einer ersten Vielzahl von radialen Positionen positioniert ist;
Berechnen einer zweiten Vielzahl von radialen Positionen auf Basis der Vielzahl von Strahlstrommessungen an den Phasenschlitzen; und
automatisches Neupositionieren der Phasenschlitze an der zweiten Vielzahl von radialen Positionen.

14. Verfahren nach einem der Ansprüche 6 bis 13, wobei das Verfahren durch das Steuersystem des Zyklotrons durchgeführt wird, wobei das Zyklotron eine Vielzahl von Untersystemen aufweist, darunter:
ein Supraleitermagnet-Untersystem;
ein Kryogenkühlungs-Untersystem;
ein Vakuum-Untersystem;
ein Hochfrequenz-Untersystem;
ein Vertikalablenker-Untersystem,
die Vielzahl von Phasenschlitzen,
die Vielzahl von elektrostatischen Extraktionsablenkern,
eine Radialstrahlsonde,
ein Phasenmessuntersystem und
die Ionenquelle;
wobei ein Betrieb des Zyklotrons eine Vielzahl von Betriebszuständen aufweist, wobei die Vielzahl von Betriebszuständen einer Vielzahl von Leistungszuständen der Vielzahl von Untersystemen entspricht,
wobei das Zyklotron konfiguriert ist, um die Vielzahl von Betriebszuständen wenigstens teilweise automatisch der Reihe nach durchzuführen, um das Zyklotron auf das Erzeugen und Extrahieren des Protonenstrahls vorzubereiten.

15. Verfahren nach Anspruch 14, wobei die Vielzahl von Betriebszuständen die Vielzahl von Untersystemen in einer vordefinierten Reihenfolge aktiviert.

## Revendications

1. Procédé (600) pour générer un faisceau de protons et effectuer un ajustement de courants de faisceau par :
le fait de générer un faisceau de protons à partir d'une source d'ions (203, 301), le faisceau de protons ayant un courant de faisceau ;
le fait de diriger, par l'intermédiaire d'une pluralité de modulateurs de trajet de faisceau, le faisceau de protons à travers une pluralité de modulateurs de courant de faisceau ;
le fait de réaliser une pluralité de mesures de courant de faisceau en effectuant une pluralité d'ajustements de courant de faisceau en faisant varier des tensions des modulateurs de trajet de faisceau ;
le fait d'analyser la pluralité de mesures de courant de faisceau afin de déterminer une correspondance entre les mesures de courant de faisceau et les tensions des modulateurs de trajet de faisceau ;
le fait de déterminer une pluralité de configurations pour la pluralité de modulateurs de trajet de faisceau en utilisant la correspondance entre les mesures de courant de faisceau et les tensions des modulateurs de trajet de faisceau, pour produire une pluralité de courants de faisceau souhaités ; et
le fait de produire automatiquement la pluralité de courants de faisceau souhaités en ajustant les modulateurs de trajet de faisceau afin de se conformer à la pluralité de configurations.

2. Procédé selon la revendication 1, dans lequel la pluralité de configurations comprend une pluralité de niveaux de tension qui correspondent à la pluralité de courants de faisceau souhaités.

3. Procédé selon la revendication 1 ou la revendication 2, dans lequel le fait de générer, le fait de diriger, le fait de réaliser, le fait d'analyser et le fait de produire sont effectuées dans un cyclotron (100, 200).

4. Procédé selon l'une quelconque des revendications 1 à 3, dans lequel la pluralité de modulateurs de trajet de faisceau comprend une pluralité de plaques déflectrices verticales (303, 403, 503).

5. Procédé selon l'une quelconque des revendications 1 à 4, dans lequel la pluralité de modulateurs de courant de faisceau comprend une pluralité de fentes de phase mobiles (306).

6. Procédé selon la revendication 3, pour l'initialisation de cyclotron automatique comprenant :
le fait de mesurer automatiquement une température d'une pluralité de composants dans un aimant supraconducteur compris dans le cyclotron ;
le fait de déterminer automatiquement une configuration actuelle de l'aimant supraconducteur du cyclotron sur la base de la température ;
le fait de paramétrer automatiquement une alimentation électrique d'aimant supraconducteur du cyclotron à la configuration de courant déterminée ;
le fait de générer le faisceau de protons dans la source d'ions comprise dans le cyclotron, l'aimant supraconducteur fonctionnant avec la configuration de courant déterminée ; et
le fait d'extraire le faisceau de protons.

7. Procédé selon la revendication 6, comprenant en outre le fait de surveiller le faisceau de protons extrait dans une boucle de commande de phase.

8. Procédé selon la revendication 7, comprenant en outre le fait de régler le faisceau de protons extrait surveillé dans la boucle de commande de phase.

9. Procédé selon la revendication 3, pour l'étalonnage automatique du fonctionnement du cyclotron, le procédé comprenant :
le fait de centrer automatiquement le faisceau de protons produit par le cyclotron ;
le fait de régler automatiquement une efficacité d'extraction du faisceau de protons produit par le cyclotron ; et
le fait d'affiner automatiquement des positions d'une pluralité de fentes de phase radiales comprises dans le cyclotron,
dans lequel le fait de centrer, le fait de régler et le fait d'affiner sont effectués automatiquement par un système de commande du cyclotron.

10. Procédé selon la revendication 9, dans lequel le fait de centrer automatiquement le faisceau de protons comprend :
le fait de mesurer une oscillation d'un courant du faisceau de protons afin de déterminer une précession du faisceau de protons ; et
le fait de réduire la précession du faisceau de protons.

11. Procédé selon la revendication 10, dans lequel le cyclotron comprend une première pluralité de tiges de compensation, et dans lequel le fait de réduire la précession du faisceau de protons comprend le fait d'utiliser la première pluralité de tiges de compensation afin d'ajuster une amplitude et une phase d'un premier harmonique du champ magnétique généré correspondant à l'oscillation du courant du faisceau de protons.

12. Procédé selon l'une quelconque des revendications 9 à 11, dans lequel le fait de régler automatiquement une efficacité d'extraction comprend le fait d'analyser le courant de faisceau du faisceau de protons et le fait de paramétrer à nouveau des valeurs correspondant à une deuxième pluralité de tiges de compensation et tensions dans une pluralité de déflecteurs d'extraction à des valeurs prédéfinies.

13. Procédé selon l'une quelconque des revendications 9 à 12, dans lequel le fait d'affiner automatiquement des positions pour la pluralité de fentes de phase radiales comprend :
le fait de déterminer une pluralité de mesures de courant de faisceau à la pluralité de fentes de phase, la pluralité de fentes de phase étant positionnée à une première pluralité de positions radiales ;
le fait de calculer une deuxième pluralité de positions radiales sur la base de la pluralité de mesures de courant de faisceau aux fentes de phase ; et
le fait de positionner automatiquement à nouveau les fentes de phase à la deuxième pluralité de positions radiales.

14. Procédé selon l'une quelconque des revendications 6 à 13, le procédé étant effectué par le système de commande du cyclotron, le cyclotron comprenant une pluralité de sous-systèmes, incluant :
un sous-système d'aimant supraconducteur ;
un sous-système de refroidissement cryogénique ;
un sous-système de vide ;
un sous-système de radiofréquence ;
un sous-système de déflecteur vertical,
la pluralité de fentes de phase,
la pluralité de déflecteurs d'extraction électrostatique,
une sonde à faisceau radial,
un sous-système de mesure de phase, et
la source d'ions ;
dans lequel un fonctionnement du cyclotron comprend une pluralité d'états de fonctionnement, la pluralité d'états de fonctionnement correspondant à une pluralité d'états de puissance de la pluralité de sous-systèmes,
dans lequel le cyclotron est configuré pour effectuer au moins partiellement automatiquement la pluralité d'états de fonctionnement en séquence afin de préparer le cyclotron à générer et à extraire le faisceau de protons.

15. Procédé selon la revendication 14, dans lequel la pluralité d'états de fonctionnement active la pluralité de sous-systèmes dans une séquence prédéfinie.
